# EUROPEAN PATENT APPLICATION

(11) **EP 3 296 450 A1**
(43) Date of publication of application: **21.03.2018**
(21) Application number: 16792220.2
(22) Date of filing: 13.05.2016
(51) Int. Cl.: D06F 58/10, D06F 58/28

(54) **CLOTHES TREATING DEVICE**

(30) Priority: 13.05.2015 JP 2015098501
(71) Applicant: Aqua Co., Ltd., Chiyoda-ku Tokyo 100-0005 (JP); Qingdao Haier Washing Machine Co., Ltd., Shandong 266101 (CN)
(72) Inventor: ONISHI, Katsuji, Tokyo 100-0005 (JP); NAGAI, Takayuki, Tokyo 100-0005 (JP); TANIKOSHI, Osamu, Tokyo 100-0005 (JP)
(74) Representative: Ziebig, Marlene
(86) International application number: PCT/CN2016/082109
(87) International publication number: WO 2016/180370

(57) **Abstract**

The present invention provides a clothes treating device capable of dealing with blocking and damage of a series of wind paths including an accommodating part for accommodating clothes. A clothes deodorizing device (1) includes: a bag body (10) for accommodating clothes; an ozone supply device (20) including an ozone generator (600) and a blowing fan (700), wherein ozone generated by the ozone generator (600) is delivered into the bag body (10) through flow of air by means of operation of the blowing fan (700); and a control part (910). The control part (910) can change an output voltage value output to the blowing fan (700) in a manner of enabling the blowing fan (700) to keep specified rotating speed, and obtain the output voltage value when controlling the blowing fan (700) to keep the specified rotating speed and a value of driving current flowing to the blowing fan (700), and stop the ozone generator (600) under a condition of judging that the obtained values are respectively less than a first threshold and a second threshold.

## Description

### TECHNICAL FIELD

The present invention relates to a clothes treating device for implementing treatment, such as deodorization, on clothes.

### BACKGROUND

In the past, a clothes renovating device is known. The clothes renovating device includes a storage warehouse capable of hanging clothes on a hanging rod for storage, and introduces circulating air absorbing clothes smell into an ozone deodorizer by enabling high-temperature and high-humidity air to perform internal circulation in the storage warehouse, thereby deodorizing the clothes (with reference to a patent literature 1).

### Existing Technical Literature

Patent Literature 1: Japan specifically disclosed No. 04-327900 Bulletin

### SUMMARY

### Problems to be solved by the invention

During use of the above clothes renovating device, the following condition may occur: a series of wind paths including the storage warehouse and the ozone deodorizer are blocked, causing poor flow of air in the wind paths or damage to the wind paths, so the air is easy to leak from a damage part. When the bad condition occurs, ozone may be continuously generated fruitlessly in a state of not ensuring performance of moderate clothes deodorization through ozone.

The present invention is a technical solution completed in view of the problem. A purpose of the present invention is to provide a clothes treating device capable of dealing with blocking and damage of a series of wind paths including an accommodating part for accommodating clothes.

### Solution for solving the problems

A clothes treating device in a first embodiment of the present invention includes: an accommodating part for accommodating clothes; an ozone supply part including an ozone generator and a blowing fan, wherein ozone generated by the ozone generator is delivered into the accommodating part through flow of air by means of operation of the blowing fan; and a control part for controlling operation of the ozone generator and the blowing fan. Herein, the control part can change an output voltage value output to the blowing fan in a manner of enabling the blowing fan to keep specified rotating speed, obtain at least one value of the output voltage value when controlling the blowing fan to keep the specified rotating speed and a value of driving current flowing through the blowing fan, and execute treatment corresponding to a judgment result under a condition of judging that the obtained value is less than a specified threshold corresponding to the value.

When a series of wind paths from the ozone supply part to the accommodating part are blocked, flow of air in the wind paths is poor and resistance of flowing air on the blowing fan becomes small. Thus, the output voltage value and the driving current value when the blowing fan is controlled to keep the specified rotating speed become smaller.

Through the above structure, when the control part obtains at least one value of the output voltage value and the driving current value and judges that the obtained values are less than thresholds corresponding to the values due to blocking of a series of wind paths, treatment corresponding to this is performed. Therefore, a clothes treating device capable of dealing with blocking of a series of wind paths can be realized.

In addition, through the above structure, since the structure is designed to detect the blocking of the wind paths through the blowing fan included in the clothes treating device, a special-purpose detecting unit for detecting the blocking of the wind paths is not required to be arranged, thereby inhibiting increase of a number of components and increase of cost.

the clothes treating device in the present embodiment can adopt the following composition: the control part obtains both of the output voltage value and the driving current value when controlling the blowing fan to keep the specified rotating speed, and executes treatment corresponding to a judgment result under a condition of judging that the obtained output voltage value is less than a first threshold and the obtained driving current value is less than a second threshold.

Through the above structure, since the wind paths are regarded as being blocked under a condition that the output voltage value is less than the first threshold and the driving current value is less than the second threshold, it is difficult to wrongly judge blocking of the wind paths due to influences of noise and the like.

A clothes treating device in a second embodiment of the present invention includes: an accommodating part for accommodating clothes; an ozone supply part including an ozone generator and a blowing fan, wherein ozone generated by the ozone generator is delivered into the accommodating part through flow of air by means of operation of the blowing fan; and a control part for controlling operation of the ozone generator and the blowing fan. Herein, the control part can change an output voltage value output to the blowing fan in a manner of enabling the blowing fan to keep specified rotating speed, obtain at least one value of the output voltage value when controlling the blowing fan to keep the specified rotating speed and a value of driving current flowing to the blowing fan, and execute treatment corresponding to a judgment result under a condition of judging that the obtained value is greater than a specified threshold corresponding to the value.

When a series of wind paths from the ozone supply part to the accommodating part are damaged to cause air is easy to leak, the air becomes easy to flow in the wind paths and resistance of flowing air on the blowing fan becomes larger. Thus, the output voltage value and the driving current value when the blowing fan is controlled to keep the specified rotating speed become larger.

Through the above structure, when the control part obtains at least one value of the output voltage value and the driving current value and judges that the obtained values are greater than thresholds corresponding to the values due to damage of a series of wind paths, treatment corresponding to this is performed. Therefore, a clothes treating device capable of dealing with damage of a series of wind paths can be realized.

In addition, through the above structure, since the structure is designed to detect the damage of the wind paths through the blowing fan included in the clothes treating device, a special-purpose detecting unit for detecting the damage of the wind paths is not required to be arranged, thereby inhibiting increase of a number of components and increase of cost.

The clothes treating device in the present embodiment can adopt the following composition: the control part obtains both of the output voltage value and the driving current value when controlling the blowing fan to keep the specified rotating speed, and executes treatment corresponding to a judgment result under a condition of judging that the obtained output voltage value is greater than a first threshold and the obtained driving current value is greater than a second threshold.

Through the above structure, since the wind paths are regarded as being damaged under a condition that the output voltage value is greater than the first threshold and the driving current value is greater than the second threshold, it is difficult to wrongly judge damage of the wind paths due to influences of noise and the like.

The clothes treating device in the present embodiment can adopt a structure which also includes a detection part. The detection part is used for detecting whether a throwing inlet for clothes arranged in the accommodating part is locked. In this case, the control part does not enable the ozone generator and the blowing fan to operate as long as the detection part does not detect that the throwing inlet is locked.

Through the above structure, a condition that damage of the wind paths is misjudged due to opening of the throwing inlet can be prevented.

The clothes treating devices in the first embodiment and the second embodiment can adopt the following composition: as a treatment corresponding to the judging result, the control part stops the ozone generator or reduces an output of the ozone generator.

Through the above structure, ozone can be prevented from being continuously generated fruitlessly when it is difficult to perform right clothes deodorization.

The clothes treating devices in the first embodiment and the second embodiment can adopt a structure which also includes an informing part for informing to the outside. In this case, as a treatment corresponding to the judging result, the control part enables the informing part to operate.

Through the above structure, blocking and damage of the wind paths can be informed to a user.

### Effects of the invention

Through the present invention, a clothes treating device can be provided, capable of dealing with blocking and damage of a series of wind paths including an accommodating part for accommodating clothes.

Effects and significance of the present invention can be further clarified by describing embodiments shown below. However, the following embodiments are just illustration during implementation of the present invention. The present invention is not limited by invention in the following embodiments.

### BRIEF DESCRIPTION OF DRAWINGS

Fig. 1 is a structural diagram illustrating a clothes deodorizing device involved in embodiments;
Fig. 2 is a structural diagram illustrating a bag body, an induction pipe and a detection lock involved in embodiments;
Fig. 3 is a structural diagram illustrating an exhaust and clothes rack retention unit involved in embodiments;
Fig. 4 is a structural diagram illustrating an ozone supply device involved in embodiments;
Fig. 5 is a structural diagram illustrating an ozone supply device involved in embodiments;
Fig. 6 is a structural diagram illustrating an ozone supply device involved in embodiments;
Fig. 7 is a structural diagram illustrating an ozone supply device involved in embodiments;
Fig. 8 is a diagram illustrating connection of an induction pipe to an ozone supply device and connection detection through a pipe detection part involved in embodiments;
Fig. 9 is a diagram illustrating an action that a lock detection part detects that a throwing inlet of a bag body is locked by a zipper involved in embodiments;
Fig. 10 is a diagram illustrating an action that a lock detection part detects that a throwing inlet of a bag body is locked by a zipper involved in embodiments;
Fig. 11 is a structural diagram illustrating a fragrance supply unit involved in embodiments;
Fig. 12 is a block diagram illustrating an ozone supply device of a structure of a control unit involved in embodiments;
Fig. 13 is a flow chart illustrating control treatment of deodorization operation involved in embodiments;
Fig. 14 is a flow chart illustrating stable control of rotating speed of a fan involved in embodiments; and
Fig. 15 is a flow chart illustrating treatment of judging blocking of wind paths and treatment of judging damage of wind paths involved in embodiments.

### DETAILED DESCRIPTION

An embodiment related to a clothes treating device of the present invention, i.e., a clothes deodorizing device, is described below with reference to drawings.

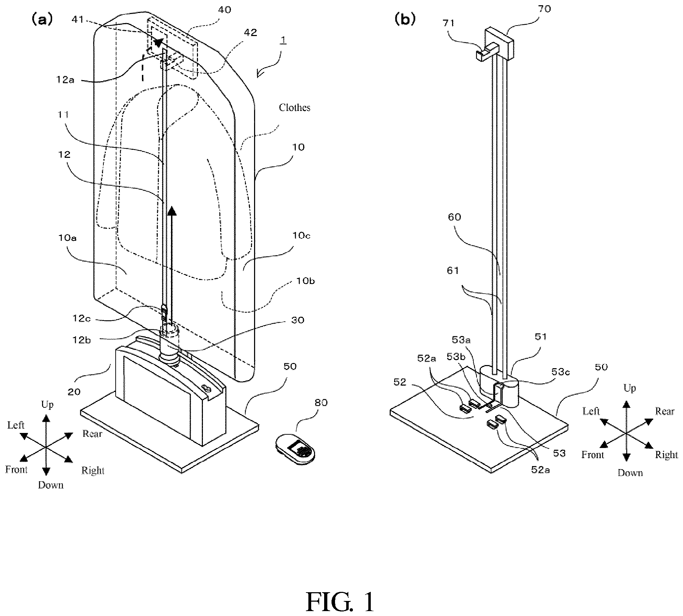

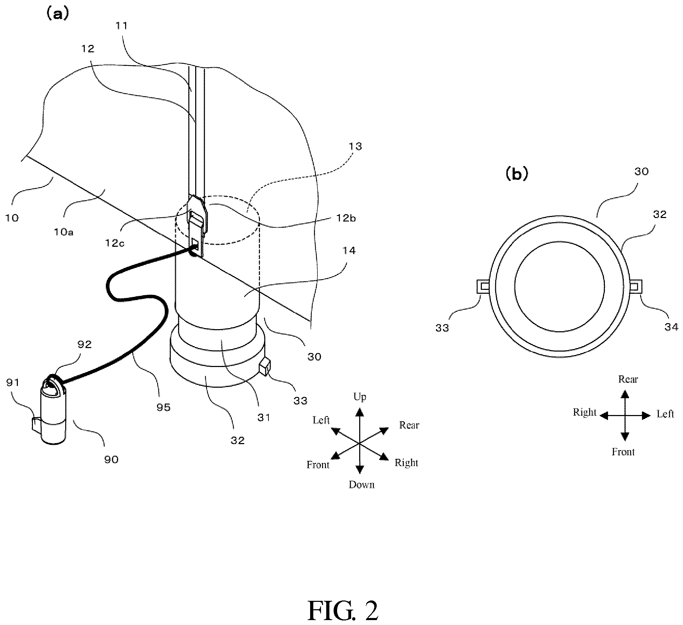

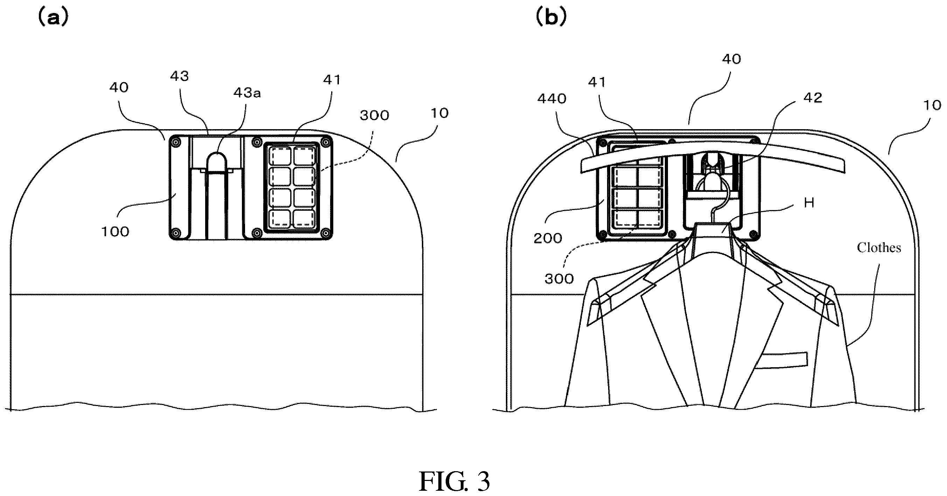

Fig. 1 is a structural diagram illustrating a clothes deodorizing device 1. Fig. 1(a) is a three-dimensional diagram illustrating a clothes deodorizing device 1. Fig. 1(b) is a three-dimensional diagram illustrating a base 50, a supporting column 60 and a bag body retention part 70 that form a clothes deodorizing device 1. In addition, Fig. 2 is a structural diagram illustrating a bag body 10, an induction pipe 30 and a detection lock 90. Fig. 2(a) is a three-dimensional diagram illustrating a lower central part of a bag body 10. Fig. 2(b) is a bottom view illustrating an induction pipe 30. Moreover, Fig. 3 is a structural diagram illustrating an exhaust and clothes rack retention unit 40. Figs. 3(a) and (b) are respectively a rear view and a main view illustrating an upper part of a bag body 10 provided with an exhaust and clothes rack retention unit 40. It should be noted that a front surface of the bag body 10 is omitted in Fig. 3(b).

By referring to Fig. 1 to Fig. 3, the clothes deodorizing device 1 includes: the bag body 10, an ozone supply device 20, an induction pope 30, an exhaust and clothes rack retention unit 40, a base 50, a supporting column 60, a bag body retention part 70 and a fragrance supply unit 80.

The bag body 10 accommodates various clothes such as western-style clothes, coats and the like. The bag body 10 is formed in a manner of overlapping a plurality of fabrics without air permeability so that tightness is adequate. The bag body 10 has an approximately lengthwise rectangular shape with flat front and rear, and an up-down size is set as a size capable of accommodating long shirts, long coats and other long clothes. In addition, a front-rear size of the bag body 10 is set as a size capable of accommodating one piece of clothes. It should be noted that the up-down size of the bag body 10 can also be set as a size incapable of accommodating long clothes, and the front-rear size can also be set as a size capable of accommodating about two or three pieces of clothes arranged in front and behind. The bag body 10 is equivalent to the accommodating part of the present invention.

In a front surface of the bag body 10, in an approximate center of a left-right direction, a gap that forms a throwing inlet 11 of the clothes is formed from an upper end to a lower end. A zipper 12 is installed at the throwing inlet 11. A starting end part 12a and an end part 12b when the zipper 12 performs locking are respectively located on an upper end and a lower end of the bag body 10. A slider 12c of the zipper 12 moves between the starting end part 12a and the end part 12b. When the slider 12c is pulled downwards from the starting end part 12a, the zipper 12 is closed so that the throwing inlet 11 is locked; and when the slider 12c is pulled upwards from the end part 12b, the zipper 12 is opened so that the throwing inlet 11 is opened.

As shown in Fig. 2(a), the detection lock 90 is connected with the slider 12c, more specifically with a handle of the slider 12c through a connecting rope 95. The detection lock 90 is used for detecting locking of the throwing inlet 11 by the zipper 12. The detection lock 90 has a cylindrical shape. A protruding part 91 is formed at a lower end part of a circumferential surface of the detection lock 90, and a hanging ring part 92 for fixing the connecting rope 95 is formed at an upper end part of the detection lock 90. One end part of the connecting rope 95 is connected with the handle of the slider 12c, and the other end part is connected with the hanging ring part 92. The connecting rope 95 may be a rope with a predetermined length, and for example, is realized by a silk ribbon, a chain, a metal wire and the like.

The ozone supply device 20 performs deodorization operation for deodorizing the clothes and fragrance increasing operation for increasing fragrance on the clothes. During deodorization operation, the ozone supply device 20 performs an action of enabling exhausted air to contain ozone so as to supply air with the ozone to the bag body 10. In addition, when the ozone supply device 20 performs fragrance increasing operation, the ozone supply device 20 performs an action of enabling the exhausted air not to contain the ozone so as to supply air without the ozone to the bag body 10. The ozone supply device 20 is equivalent to an ozone supply part of the present invention.

The induction pipe 30 is connected with the bag body 10 and the ozone supply device 20. The air exhausted from the ozone supply device 20 is guided into the bag body 10. As shown in Fig. 2, an air inlet 13 is arranged in a central part of a lower surface on the bag body 10; and a top end part of the induction pipe 30 is fixed to a cylindrical part 14 which droops from the inlet 13. The induction pipe 30 includes a cylindrical main part 31 with a relatively small outer diameter and a cylindrical connecting part 32 formed under the main part 31 and having a relatively large outer diameter. A right claw part 33 and a left claw part 34 are respectively formed at a lower end of the connecting part 32 and in a position facing a right side and a left side of the bag body 10 in a state of being installed on the bag body 10.

An exhaust and clothes rack retention unit 40 is arranged on an upper part of a rear surface of the bag body 10. As shown in Fig. 3, the exhaust and clothes rack retention unit 40 includes an exhaust part 41, a clothes rack retention part 42 and an installation part 43. The exhaust and clothes rack retention unit 40 is composed of a rear unit 100 and a front unit 200 connected in a manner of clamping the upper part of the rear surface of the bag body 10 from an outer side and an inner side.

The air from interior of the bag body 10 is exhausted to the outside through the exhaust part 41. An ozone removing filter 300 is installed on the exhaust part 41. The ozone removing filter 300 uses, for example, an activated carbon/catalyst filter formed by transferring activated carbon and a catalyst to base material such as aluminum. The ozone removing filter 300 removes the ozone included in the air through the exhaust part 41. The clothes rack retention part 42 retains and hangs a clothes rack H for clothes. In addition, the clothes rack retention part 42 retains an upper mask 440. The upper mask 440 is formed in a lalongate platy shape slightly bent into an arch. The upper surface of the bag body 10 is strengthened from an inner side through the upper mask 440. The installation part 43 is formed in a square box shape that extends rearwards, and an installation hole 43a is formed in a rear surface of the installation part 43.

The base 50 is a flat plate with a specified shape, such as a quadrangle. The ozone supply device 20 is carried on the base 50. A supporting part 51 for supporting the supporting column 60 is formed at a rear of the base 50. Moreover, at the base 50, a first fixing part 52 and a second fixing part 53 for fixing the ozone supply device 20 in a manner of enabling the front surface of the ozone supply device 20 to face a direction of the front surface of the base 50 are formed. The first fixing part 52 is composed of a plurality of hooked claw parts 52a. The second fixing part 53 includes: an L-shaped elastic rod 53a with one end part supported by the base 50, a bulge 53b formed near the rear slightly relative to one end part of the elastic rod 53a, and a pressing part 53c formed on the other end part of the elastic rod 53a. When the pressing part 53c is pressed downwards, the elastic rod 53a generates elastic deformation and the bulge 53b is contracted into the lower part.

The supporting column 60 is composed of two rods 61. A lower end part of the supporting column 60 is installed on the supporting part 51, and is erect relative to the base 50. The supporting column 60 may be not composed of two rods 61, but composed of one rod or more than three rods. In addition, a telescopic mechanism capable of adjusting the height of the supporting column 60 can also be arranged on the supporting column 60.

A bag body retention part 70 is installed at an upper end of the supporting column 60. The bag body retention part 70 has a retention part 71 which protrudes forwards. By inserting the retention part 71 into the installation hole 43a of the installation part 43, the bag body 10 can be hanged and retained in a manner of not moving in any direction of front and rear, up and down and left and right.

The fragrance supply unit 80 is used when fragrance increasing operation is performed through the ozone supply device 20. The fragrance supply unit 80 is detachably installed on the induction pipe 30, so that air supplied to the bag body 10 contains fragrant ingredients.

Next, a detailed structure of the ozone supply device 20 is described.

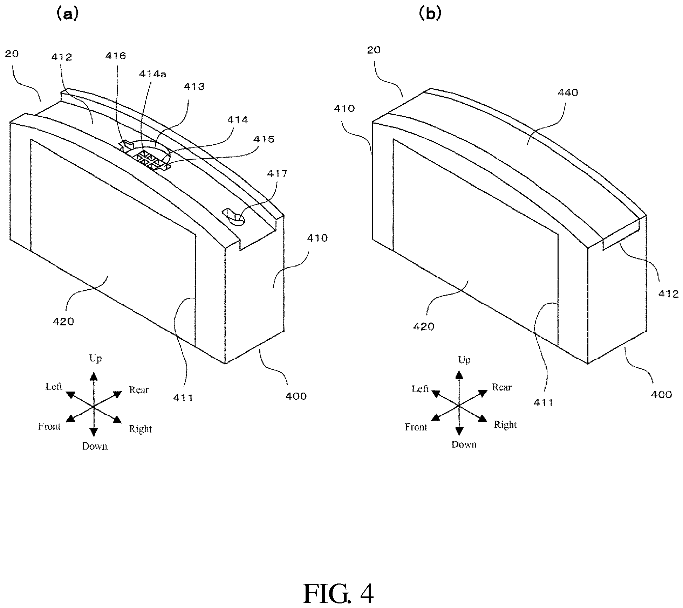

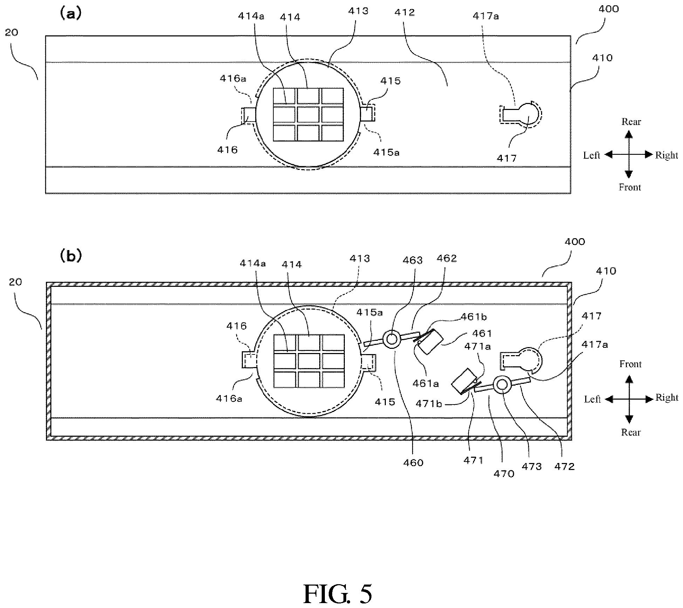

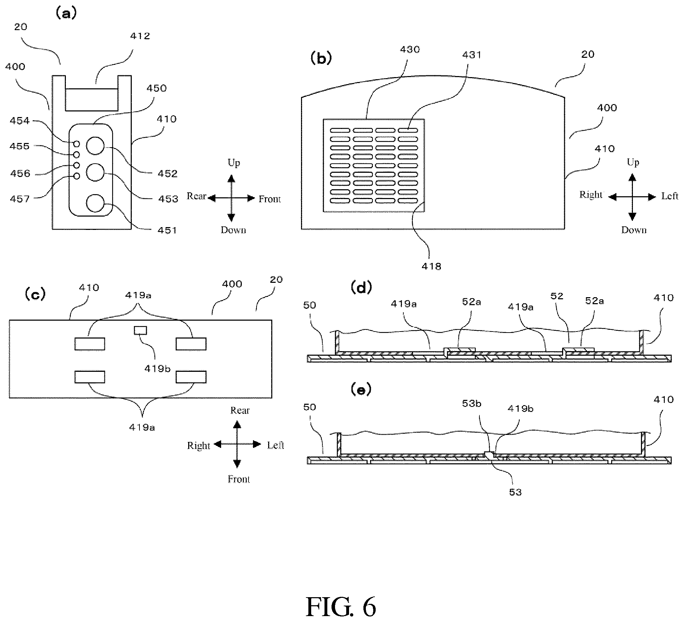

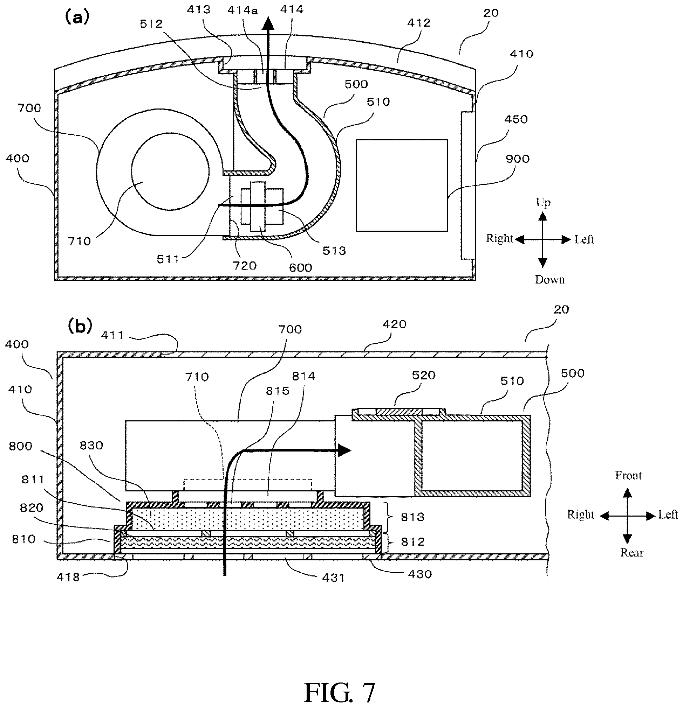

Fig. 4 to Fig. 7 are structural diagrams illustrating an ozone supply device 20. Fig. 4(a) is a three-dimensional diagram illustrating an ozone supply device 20 in a state without installing an upper mask 440. Fig. 4(b) is a three-dimensional diagram illustrating an ozone supply device 20 in a state with an upper mask 440. Fig. 5(a) is a top view illustrating an ozone supply device 20. Fig. 5(b) is a transverse section view for observing an upper surface of a shell 400 of an ozone supply device 20 from an inner side. Figs. 6(a) to (c) are a left view, a rear view and a bottom view illustrating an ozone supply device 20. Figs. 6(d) and (e) are side section views illustrating a main part of an ozone supply device 20 in a state of being fixed to the base 50. Fig. 7(a) is a longitudinal section view for observing an ozone supply device 20 from a rear. Fig. 7(b) is a transverse section view for overlooking a main part of an ozone supply device 20. It should be noted that a right inserting concave part 415, a left inserting concave part 416, a lock inserting concave part 417, a pipe detection part 460 and a lock detection part 470 are not shown in Fig. 7(a).

The ozone supply device 20 includes a shell 400, a vent pipe 500, an ozone generator 600, a blowing fan 700, an air suction unit 800 and a control unit 900.

The shell 400 includes: a shell body 410, a front mask 420, an air suction hood 430, an upper mask 440 and an operation part 450. As shown in Fig. 4(a), the shell body 410 has a lalongate rectangular shape with an upper surface gently bent. A front surface opening part 411 is formed on a front surface of the shell body 410. The front surface opening part 411 is detachable locked through the front mask 420.

On the upper surface of the shell body 410, a concave part 412 sunk into a shape identical with a shape of the upper mask 440 is formed. An inserting port part 413 sunk into a circular shape is arranged in the center of the concave part 412. An exhaust port 414 with a latticed rectification rib 414a is formed in the inserting port part 413. At the concave part 412, a right inserting concave part 415 and a left inserting concave part 416 with shapes corresponding to the right claw part 33 and the left claw part 34 of the induction pipe 30 are formed on the left side and the right side of the inserting port part 413. As shown in Figs. 5(a) and (b), at a front side of the right inserting concave part 415, a right opening part 415a is formed in such a manner that the right claw part 33 inserted into the right inserting concave part 415 only moves to a right turning direction by about an amount of one right claw part 33. Similarly, at a rear side of the left inserting concave part 416, a left opening part 416a is formed in such a manner that the left claw part 34 inserted into the left inserting concave part 416 only moves to a right turning direction by about an amount of one left claw part 34.

At the concave part 412, the lock inserting concave part 417 with a shape corresponding to the detection lock 90 is formed at a right end part. As shown in Figs. 5(a) and (b), an opening part 417a is formed in a side surface of the rear side of the lock inserting concave part 417.

As shown in Fig. 4(b), when the clothes deodorizing device 1 is not used, the upper mask 440 removed from the bag body 10 can be installed on the concave part 412. Thus, since a retention place of the upper mask 440 removed from the bag body 10 is ensured, the upper mask 440 can be prevented from being lost when not used. In addition, dust can be prevented from entering the shell 400 from the exhaust port 414 and the lock inserting concave part 417 when the clothes deodorizing device is not used.

As shown in Fig. 5(b), the pipe detection part 460 and the lock detection part 470 are configured at an inner side of the upper surface of the shell body 410. The pipe detection part 460 includes a pipe detection switch 461 and a relay rod 462. The pipe detection switch 461 has a switch part 461a and a rod part 461b for pressing the switch part 461a. The relay rod 462 is installed in a free rotation mode on a rotating shaft 463 formed at the inner side of the upper surface of the shell body 410. One end of the relay rod 462 is located near the right inserting concave part 415, and the other end of the relay rod 462 comes into contact with the pipe detection switch 461. The lock detection part 470 includes a lock detection switch 471 and a relay rod 472. The lock detection switch 471 has a switch part 471a and a rod part 471b for pressing the switch part 471a. The relay rod 472 is installed in a free rotation mode on a rotating shaft 473 formed at the inner side of the upper surface of the shell body 410. One end of the relay rod 472 is located near the lock inserting concave part 417, and the other end of the relay rod 472 comes into contact with the lock detection switch 471. The lock detection part 470 is equivalent to the detection part of the present invention.

As shown in Fig. 6(a), the operation part 450 is arranged on a left side surface of the shell body 410. The operation part 450 includes a power button 451, a deodorization button 452 and a fragrance increasing button 453. The power button 451 is a button for switching on and switching off a power supply of the clothes deodorizing device 1. The deodorization button 452 is a button for starting deodorization operation. The fragrance increasing button 453 is a button for starting fragrance increasing operation. In addition, the operation part 450 includes a first informing part 454, a second informing part 455, a third informing part 456 and a fourth informing part 457. The first informing part 454 is, for example, composed of LED, and informs that the induction pipe 30 is not connected with the ozone supply device 20 through lighting up. The second informing part 455 is, for example, composed of LED, and informs that the throwing inlet 11 of the bag body 10 is not locked through lighting up. The third informing part 456 is, for example, composed of LED, and informs that a series of wind paths from the air suction unit 800 of the ozone supply device 20 to the exhaust part 41 of the bag body 10 are blocked through lighting up. The fourth informing part 457 is, for example, composed of LED, and informs that a series of wind paths are damaged through lighting up. The third informing part 456 and the fourth informing part 457 are equivalent to the informing parts of the present invention.

As shown in Fig. 6(b), an air suction port 418 is formed in a rear surface of the shell body 410. The air suction port 418 is detachably locked through the air suction hood 430. A plurality of air suction holes 431 are formed in the air suction hood 430.

As shown in Fig. 6(c), at a bottom surface of the shell body 410, a first installation hole 419a is formed in a position corresponding to each claw part 52a of the first fixing part 52 of the base 50. A second installation hole 419b is formed in a position corresponding to the bulge 53b of the second fixing part 53. After a user presses the pressing part 53c of the second fixing part 53 downwards to contract the bulge 53b, and when the claw part 52a loads the ozone supply device 20 on the base 50 through the first installation hole 419a so as to transversely slide the ozone supply device 20, as shown in Fig. 6(d), the claw part 52a is clamped with a bottom surface of the shell body 410. Then, when the user stops pressing the pressing part 53c, as shown in Fig. 6(e), the bulge 53b is embedded into the second installation hole 419b. Thus, the ozone supply device 20 is fixed to the base 50 in a manner of not moving in directions of up and down, front and rear and left and right. Therefore, the ozone supply device 20 can be prevented from being upside down due to a force applied to the ozone supply device 20 when the bag body 10 is inflated because of the air supplied by the ozone supply device 20.

A vent pipe 500, an ozone generator 600, a blowing fan 700, an air suction unit 800 and a control unit 900 are configured in the shell 400.

As shown in Figs. 7(a) and (b), the vent pipe 500 includes a pipe body 510 and a pipe cover 520. An induction port 511 of the pipe body 510 is connected with the exhaust port 720 of the blowing fan 700, and an eduction port 512 is connected with the exhaust port 414. The ozone generator 600 is configured near the induction port 511 in the pipe body 510. The pipe body 510 has the following shape: the pipe body 510 extends upwards to the eduction port 512 after bending in a manner of extending from the induction port 511 to the left and beginning to go back to the right over a part of the configuration position of the ozone generator 600. Namely, a part of a downstream side of the pipe body 510 forming the ozone generator 600 crawls in an S shape.

The ozone generator 600 is a discharge type ozone generator. Discharge such as corona discharge, silent discharge and the like is generated between a pair of electrodes, and ozone is generated through the air between a pair of electrodes. At a front surface of the pipe body 510, an opening part 513 is formed in a position corresponding to the ozone generator 600. The opening part 513 is locked through the pipe cover 520. The user can remove the front mask 420 and the pipe cover 520, so as to clean the electrodes through the opening part 513 to maintain the ozone generator 600.

The blowing fan 700 is a centrifugal fan. A suction inlet 710 is arranged in a side surface of the blowing fan 700, and an exhaust port 720 is arranged in a circumferential surface of the blowing fan 700. The suction inlet 710 is opposite to the air suction port 418 on the rear surface of the shell 400. The blowing fan 700 obtains the air from the suction inlet 710, and delivers the obtained air to the ozone generator 600 in the vent pipe 500. The blowing fan 700 can also use other fans besides the centrifugal fan, such as an axial flow fan.

As shown in Fig. 7(b), an air suction unit 800 is arranged between the air suction port 418 of the shell 400 and the suction inlet 710 of the blowing fan 700. The air suction unit 800 includes an air suction pipe 810, a dust filter 820 and an ozone removing filter 830.

The air suction pipe 810 is divided into a first filter accommodating part 812 at a side of the air suction port 418 and a second filter accommodating part 813 at a side of the blowing fan 700 through a latticed dividing plate 811. A dust filter 820 is accommodated at the first filter accommodating part 812, and an ozone removing filter 830 is accommodated at the second filter accommodating part 813. The dust filter 820 removes dust included in the air obtained from the air suction port 418. The ozone removing filter 830 removes the ozone included in the air passing through the dust filter 820. The ozone removing filter 830, identical with the ozone removing filter 300 of the exhaust and clothes rack retention unit 40, can use activated carbon/catalyst filter.

The air suction pipe 810 is provided with a connecting part 814 connected with the suction inlet 710 of the blowing fan 700. The connecting part 814 is connected with the second filter accommodating part 813 through a communication hole 815.

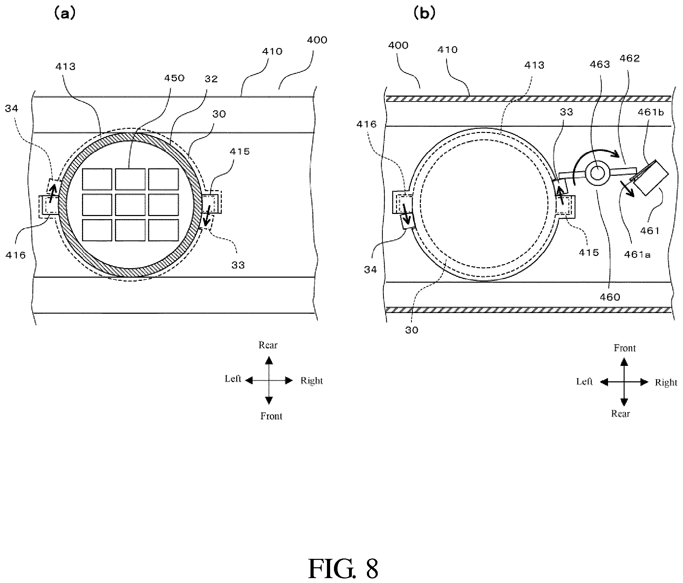

Next, by referring to Fig. 8, connection of the induction pipe 30 to the ozone supply device 20 and connection detection through the pipe detection part 460 are described.

When the induction pipe 30 is connected with the ozone supply device 20, as shown in Fig. 8(a), the connecting part 32 of the induction pipe 30 is inserted into the inserting port part 413 in a manner of respectively inserting the right claw part 33 and the left claw part 34 into the right inserting concave part 415 and the left inserting concave part 416. Then, when the induction pipe 30 is observed from the upper part and rotates to the right, the right claw part 33 and the left claw part 34 respectively move to the inner side of the upper surface of the shell body 410 through the right opening part 415a and the left opening part 416a, and are clamped with the upper surface of the shell body 410. Thus, the induction pipe 30 does not fall to the upper part.

In this way, the induction pipe 30 is connected with the ozone supply device 20. As shown in Fig. 8(b), when the right claw part 33 moves to the inner side of the upper surface of the shell body 410, one end side of the relay rod 462 is pressed by the right claw part 33. The relay rod 462 rotates; a rod part 461b is pressed by the other end side of the relay rod 462; and a switch part 461a is pressed by the pressed rod part 461b. Thus, the pipe detection switch 461 detects that the induction pipe 30 is installed on the inserting port part 413.

It should be noted that when the induction pipe 30 is removed from the inserting port part 413, the rod part 461b rotates the relay rod 462 through self elasticity, and simultaneously returns to an initial position. Thus, the pipe detection switch 461 detects that the induction pipe 30 is removed from the inserting port part 413.

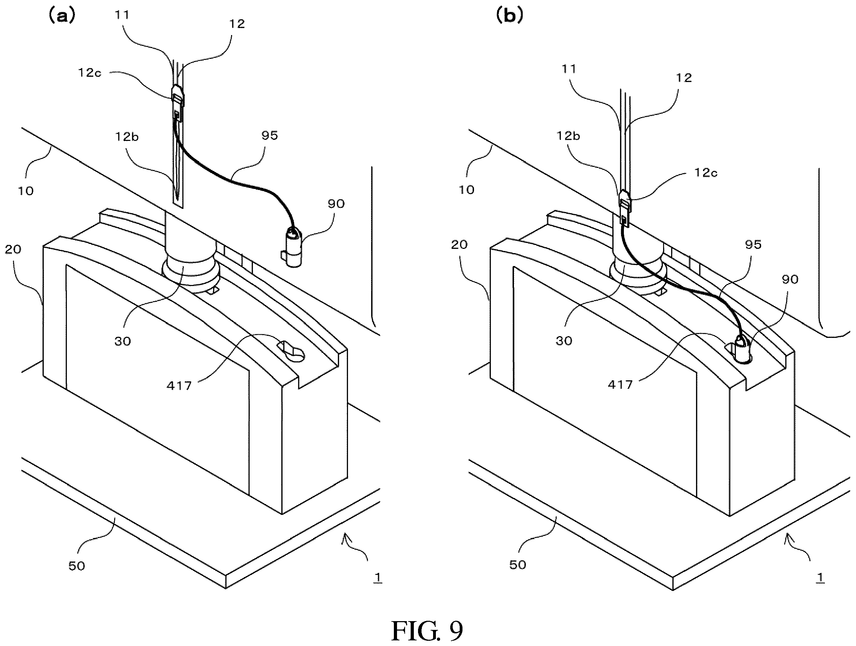

Next, by referring to Fig. 9 to Fig. 10, an action that a lock detection part 470 detects that a throwing inlet 11 of a bag body 10 is locked by a zipper 12 is described.

A connecting rope 95 for connecting the detection lock 90 and the slider 12c has a length that the detection lock 90 arrives at the lock inserting concave part 417 when the zipper 12 is locked near the end part 12b. Therefore, as shown in Fig. 9(a), when the slider 12c is not located near the end part 12b, the detection lock 90 fails to reach the lock inserting concave part 417 and the user cannot insert the detection lock 90 into the lock inserting concave part 417. Namely, at least in a state that the zipper 12 is completely pulled, the detection lock 90 fails to reach the lock inserting concave part 417. On the other hand, as shown in Fig. 9(b), in a state that the zipper 12 is completely closed, the detection lock 90 can reach the lock inserting concave part 417 and the detection lock 90 is inserted into the lock inserting concave part 417.

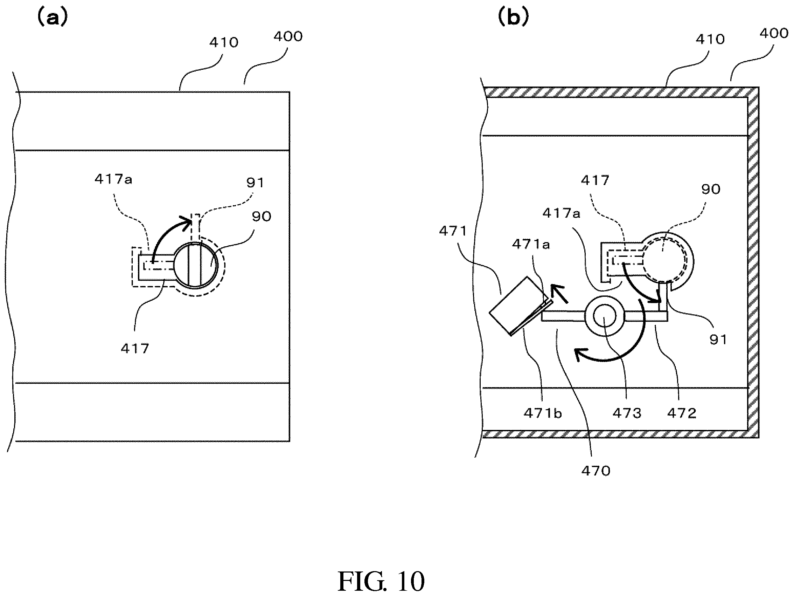

When the user locks the throwing inlet 11 to the end through the zipper 12, as shown in Fig. 10(a), the detection lock 90 is inserted into the lock inserting concave part 417 and the inserted detection lock 90 is observed from the upper part and rotates to the right. As shown in Fig. 10(b), the protruding part 91 of the detection lock 90 moves to the inner side of the upper surface of the shell body 410 through the opening part 417a, and one end side of the relay rod 472 is pressed through the moved protruding part 91. The relay rod 472 rotates; the rod part 471b is pressed through the other end side of the relay rod 472; and the switch part 471a is pressed by the pressed rod part 471b. Thus, the lock detection switch 471 detects that the detection lock 90 is inserted into the lock inserting concave part 417, i.e., detects that the throwing inlet 11 of the bag body 10 is locked by the zipper 12.

It should be noted that when the detection lock 90 is removed from the lock inserting concave part 417, the rod part 471b rotates the relay rod 472 through self elasticity, and simultaneously returns to an initial position. Thus, the lock detection switch 471 detects that the detection lock 90 is removed from the lock inserting concave part 417.

Next, a detailed structure of the fragrance supply unit 80 is described.

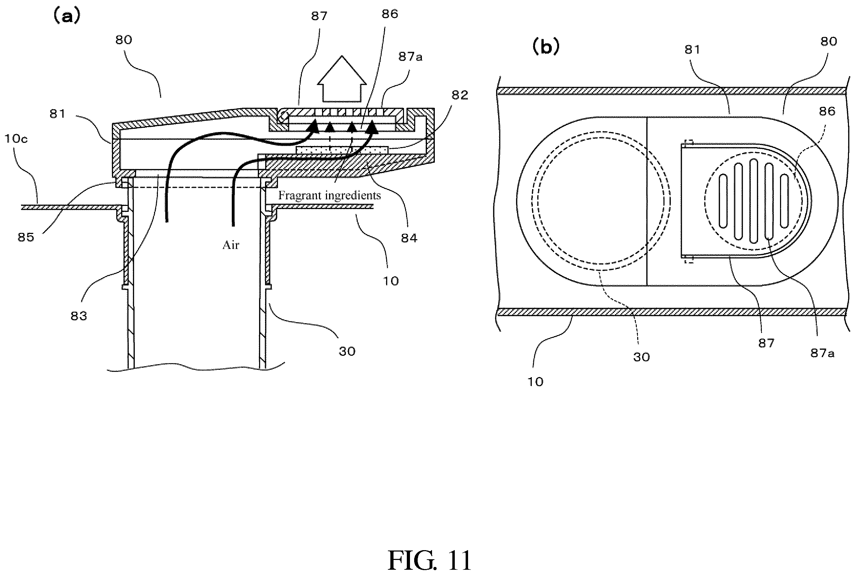

Fig. 11 is a structural diagram illustrating a fragrance supply unit 80. Figs. 11(a) and (b) are respectively a longitudinal section view and a transverse section view illustrating a central part of a lower part of a bag body 10 in a state that a fragrance supply unit 80 is installed on an induction pipe 30.

The fragrance supply unit 80 includes an accommodating box 81 which has an oval box shape when overlooked, and a fragrant body 82 accommodated in the accommodating box 81.

At a bottom surface of the accommodating box 81, an air suction port 83 is formed, and a loading part 84 adjacent to the air suction port 83 and composed of a plurality of ribs extending to a long edge direction is formed. The fragrant body 82 is loaded above the loading part 84. In addition, at the bottom surface of the accommodating box 81, a cylindrical connecting port 85 is formed in a manner of encircling the air suction port 83.

At an upper surface of the accommodating box 81, an opening part 86 is formed in a position directly above the loading part 84. The opening part 86 is covered by a cover part 87 with a plurality of vent holes 87a in an openable and closable manner. The fragrant body 82 is formed by porous material and the like which can be immersed in a liquid flavoring agent. The user opens the cover part 87 and arranges the fragrant body 82 into the accommodating box 81 from the opening part 86.

Next, a structure of the control unit 900 is described.

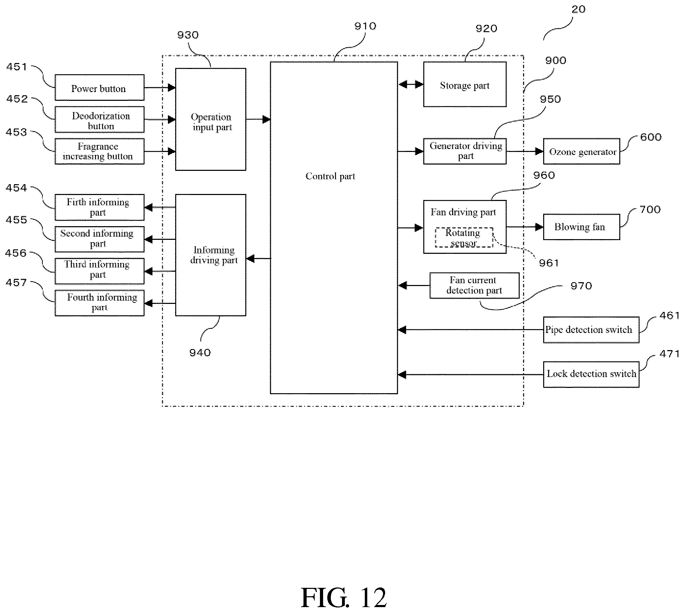

Fig. 12 is a block diagram illustrating an ozone supply device 20 of a structure of a control unit 900. The control unit 900 includes: a control part 910, a storage part 920, an operation input part 930, an informing driving part 940, a generator driving part 950, a fan driving part 960 and a fan current detection part 970.

The storage part 920 includes an EEPROM, a RAM and the like. The storage part 920 stores programs for executing deodorization operation and fragrance increasing operation. In addition, the storage part 920 stores various parameters and various control marks for execution the programs.

The control marks include an arrival mark, a blocking mark and a damage mark. The arrival mark is used for judging whether a rotating speed of the blowing fan 700 is equal to a preset target rotating speed. The blocking mark is used for judging whether a series of wind paths from the air suction unit 800 of the ozone supply device 20 to the exhaust part 41 of the bag body 10 are blocked. The damage mark is used for judging whether a series of wind paths are damaged. It should be noted that the target rotating speed may not a numerical value, and may be values with a certain degree of range.

The operation input part 930 outputs an input signal corresponding to a button operated by the user in a power button 451, a deodorization button 452 and a fragrance increasing button 453 to the control part 910. The informing driving part 940 drives a first informing part 454, a second informing part 455, a third informing part 456 and a fourth informing part 457 according to a control signal from the control part 910. The generator driving part 950 drives the ozone generator 600 according to a control signal from the control part 910.

The fan driving part 960 drives the blowing fan 700 according to a control signal from the control part 910. The fan driving part 960 includes a rotation sensor 961 for detecting the rotating speed of the blowing fan 700, and outputs an output voltage of a value corresponding to the rotating speed detected by the rotation sensor 961 to the blowing fan 700 in a manner of enabling the blowing fan 700 to rotate at the target rotating speed. For example, drive control can be realized by using PWM. In this case, a duty ratio is determined by the control part 910 according to the detected rotating speed, and a pulse voltage of a pulse width corresponding to the determined duty ratio is output from the fan driving part 960 to the blowing fan 700.

The fan current detection part 970 detects a value of driving current flowing through the blowing fan 700 driven by the fan driving part 960, and outputs a detection signal corresponding to the detected driving current value to the control part 910.

When the pipe detection switch 461 detects that the induction pipe 30 is installed on the ozone supply device 20, the detection signal corresponding thereto is output to the control part 910. When the lock detection switch 471 detects that the detection lock 90 is installed on the lock inserting concave part 417, i.e., when the throwing inlet 11 of the bag body 10 is locked by the zipper 12, the detection signal corresponding thereto is output to the control part 910.

The control part 910 controls the informing driving part 940, the generator driving part 950, the fan driving part 960 and the like according to signals from the operation input part 930, the fan current detection part 970, the pipe detection switch 461, the lock detection switch 471 and the like in accordance with the programs stored in the storage part 920.

Next, deodorization operation and fragrance increasing operation of the clothes deodorizing device 1 are described.

Under a condition of performing deodorization operation, the user accommodates the clothes hanged on the clothes rack H for clothes into the bag body 10 hanged on the bag body retention part 70. At this moment, as shown in Fig. 3(b), the clothes rack H for clothes is hanged on the clothes rack retention part 42 in the bag body 10. In this way, in the bag body 10, the clothes are hanged through the clothes rack retention part 42. The user presses the deodorization button 452 of the operation part 450 of the ozone supply device 20.

When deodorization operation is started, the air suction pipe 810 obtains outside air from the air suction port 418, and removes dust and ozone included in the air through the dust filter 820 and the ozone removing filter 830 in the air suction pipe 810. The air without dust and ozone is delivered into the vent pipe 500 through the blowing fan 700 (with reference to an arrow in Fig. 7(b)). The air flowing through the vent pipe 500 is mixed with the ozone generated by the ozone generator 600 when passing through the ozone generator 600. In this way, air with the ozone arrives at the exhaust port 414 through the vent pipe 500 and is exhausted from the exhaust port 414 (with reference to an arrow in Fig. 7(a)).

The air exhausted from the ozone supply device 20 and including the ozone is guided into the bag body 10 through the induction pipe 30. As shown by an arrow in Fig. 1(a), the air guided into the bag body 10 and including the ozone is in contact with the clothes in the bag body 10 from bottom to top and simultaneously flows. The clothes are deodorized through a deodorization effect of the ozone included in the air.

The air with a reduced ozone concentration through deodorization for the clothes, as shown by a dotted arrow in Fig. 1(a), is exhausted out of the bag body 10 through the exhaust part 41 above the bag body 10. Ozone is removed from deodorized air through the ozone removing filter 300 when the deodorized air passes through the exhaust part 41. Thus, the concentration of the ozone in the air exhausted from the bag body 10 is further reduced.

Next, under a condition of performing fragrance increasing operation, the user accommodates the clothes into the bag body 10 hanged on the bag body retention part 70, and as shown in Fig. 11, in the bag body 10, the fragrance supply unit 80 provided with the fragrant body 82 is installed on the induction pipe 30. The user presses the fragrance increasing button 453 of the operation part 450 of the ozone supply device 20.

When the fragrance increasing operation is started, as shown in Fig. 11(a), the air exhausted from the induction pipe 30 is introduced into the accommodating box 81 from the air suction port 83a. The introduced air passes through the fragrant body 82. Fragrant ingredients included in the fragrant body 82 volatilize, and are mixed into the air. The air with the fragrant ingredients is exhausted into the bag body 10 through the opening part 86 and the vent hole 87a.

The air with the fragrant ingredients flows from bottom to top in the bag body 10, and is exhausted from the exhaust part 41 to an outer part of the bag body 10. The fragrant ingredients are absorbed to the clothes accommodated in the bag body 10.

In the present embodiment, in deodorization operation, whether a series of wind paths from the air suction unit 800 of the ozone supply device 20 to the exhaust part 41 of the bag body 10 (called as "a series of wind paths" for short below) in the clothes deodorizing device 1 are blocked and damaged can be monitored by executing treatment for judging blocking of the wind paths and treatment of judging damage of the wind paths. Moreover, under a condition that a series of wind paths are blocked and damaged, the ozone generator 600 is stopped, and the third informing part 456 and the fourth informing part 457 light up.

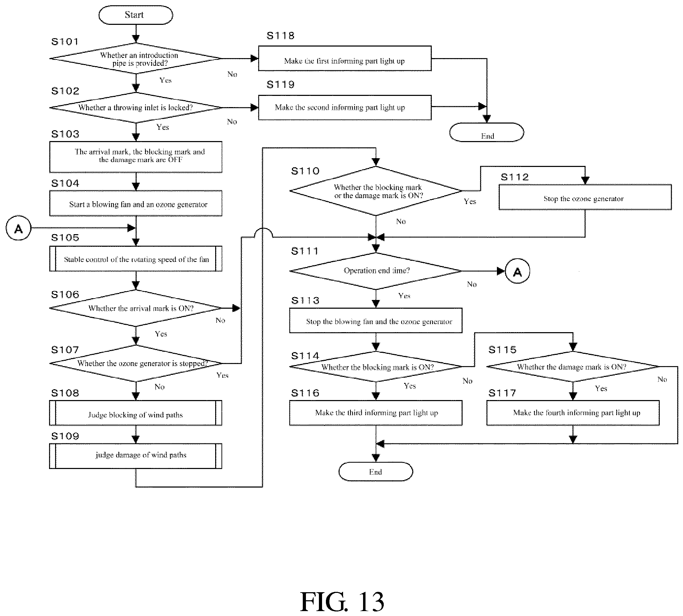

Fig. 13 is a flow chart illustrating control treatment of deodorization operation. A control action of deodorization operation including treatment of judging blocking of wind paths and treatment of judging damage of wind paths is described below according to the flow chart of Fig. 13.

When deodorization operation is started, the control part 910 judges whether the inserting port part 413 is provided with the induction pipe 30 (S101) according to a detection signal from the pipe detection switch 461. In addition, the control part 910 judges whether the throwing inlet 11 of the bag body 10 is locked (S102) according to a detection signal from the lock detection switch 471.

The induction pipe 30 is installed on the inserting port part 413, and under a condition that the throwing inlet 11 of the bag body 10 is locked (S101: Yes->S102: Yes), the control part 910 starts the blowing fan 700 and the ozone generator 600(S104) after setting the arrival mark, the blocking mark and the damage mark as OFF states (S103).

When the blowing fan 700 is started, the control part 910 performs treatment for stable control of the rotating speed of the fan to enable the blowing fan 700 to rotate at the target rotating speed.

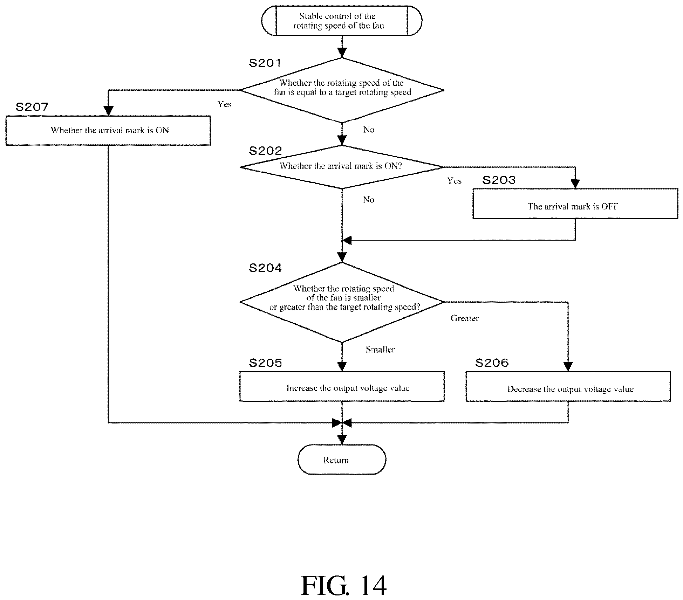

Fig. 14 is a flow chart illustrating stable control of the rotating speed of a fan. By referring to Fig. 14, stable control of the rotating speed of the fan is described.

The control part 910 judges whether the rotating speed of the blowing fan 700 is equal to the target rotating speed (S201). Under a condition that the rotating speed of the blowing fan 700 deviates from the target rotating speed (S201: No), the control part 910 sets the arrival mark as an OFF state (S203) as long as the arrival mark is in an ON state (S202: Yes). Next, the control part 910 judges whether the rotating speed of the blowing fan 700 is smaller or larger relative to the target rotating speed. When the rotating speed of the blowing fan 700 is smaller (S204: smaller), the fan driving part 960 is controlled to increase a voltage value output to the blowing fan 700 (S205); and when the rotating speed of the blowing fan 700 is larger (S204: larger), the fan driving part 960 is controlled to decrease an output value of the fan (S206). For example, under a condition of PWM control, the fan driving part 960 increases the pulse width of pulse voltage so as to increase the output voltage value, and decreases the pulse width so as to decrease the output voltage value.

On the other hand, in step S201, when the rotating speed of the blowing fan 700 is judged to be equal to the target rotating speed (S201: Yes), the control part 910 sets the arrival mark as an ON state (S207). At this moment, the control part 910 does not increase or decrease the voltage value output to the blowing fan 700.

In this way, through stable control of the rotating speed of the fan, the blowing fan 700 rotates in a manner of keeping the target rotating speed. During a period of keeping the target rotating speed, the arrival mark keeps the ON state.

By returning to Fig. 13, the control part 910 judges whether the arrival mark is in the ON state (S106), and judges whether the ozone generator 600 is stopped (S107). When the arrival mark is in the ON state (S106: Yes) and the ozone generator 600 is not stopped (S107: No), the control part 910 executes treatment of judging blocking of wind paths (S108) and treatment of judging damage of wind paths (S109). It should be noted that when the arrival mark is not in the ON state (S106: No) or the ozone generator 600 is stopped (S107: Yes), the control part 910 does not execute treatment of judging blocking of wind paths and treatment of judging damage of wind paths.

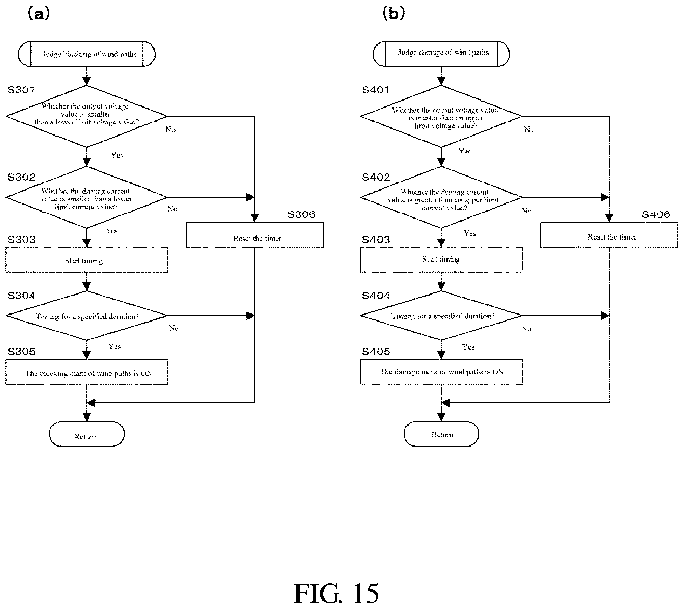

Fig. 15(a) is a flow chart illustrating treatment of judging blocking of wind paths. Fig. 15(b) is a flow chart illustrating treatment of judging damage of wind paths.

Firstly, by referring to Fig. 15(a), treatment of judging blocking of wind paths is described. Assuming a condition that part of a series of wind paths are blocked in the clothes deodorizing device 1 in a deodorization operation process. For example, assuming a condition that the air suction pipe 810 is blocked due to clogging of the dust filter 820 and the ozone removing filter 830 of the ozone supply device 20, and a condition that the exhaust part 41 is blocked due to clogging of the ozone removing filter 300 of the bag body 10. When a series of wind paths are blocked, the resistance during rotation of the blowing fan 700 decreases a reduced amount of air delivery amount of the blowing fan 700, as long as driving power for driving the blowing fan 700 is small. Therefore, it is easy to cause that the output voltage value when the blowing fan 700 keeps the target rotating speed is lower than a normal range, and it is easy to cause that the driving current value of the blowing fan 700 is also lower than the normal range. Therefore, a lower limit voltage value that indicates a lower limit of the normal range of the output voltage value and a lower limit current value that indicates a lower limit of the normal range of the driving current value of the blowing fan 700 are determined in advance according to experiments and the like, and are stored in the storage part 920.

The control part 910 judges whether the output voltage value is lower than the lower limit voltage value, and whether the driving current value of the blowing fan 700 is lower than the lower limit current value (S301, S302).

When the output voltage value is lower than the lower limit voltage value, and the driving current value is lower than the lower limit current value (S301: Yes->S302: Yes), the control part 910 starts timing through a timer (S303). Then, when the timer times to a specified duration, such as 5 seconds (S304: Yes), i.e., when a state that the output voltage value is lower than the lower limit voltage value and the driving current value is lower than the lower limit current value at least lasts for the specified duration, the control part 910 sets the blocking mark as an ON state (S305). Before the timer times to the specified duration, when the output voltage value is greater than the lower limit voltage value or the driving current value is greater than the lower limit current value, the control part 910 resets the timer (S306).

In this way, under a condition that a series of wind paths are blocked, the blocking mark is set as an ON state through treatment of judging blocking of the wind paths.

Next, by referring to Fig. 15(b), treatment of judging damage of the wind paths is described. Assuming a condition that part of a series of wind paths are damaged in the clothes deodorizing device 1 in a deodorization operation process. For example, assuming a condition that the induction pipe 30 is broken and the bag body 10 is damaged. When a series of wind paths are damaged and air is easy to leak from the wind paths, the air becomes easy to flow in the wind paths so that the air delivery amount of the blowing fan 700 is increased; and the resistance during rotation of the blowing fan 700 is increased by a corresponding amount, causing that the blowing fan 700 needs large driving power. Therefore, the output voltage value for enabling the blowing fan 700 to keep the target rotating speed is easy to exceed the normal range, the driving current value of the blowing fan 700 is also easy to exceed the normal range. Therefore, an upper limit voltage value that indicates an upper limit of the normal range of the output voltage value and an upper limit current value that indicates an upper limit of the normal range of the driving current value of the blowing fan 700 are determined in advance according to experiments and the like, and are stored in the storage part 920.

The control part 910 judges whether the output voltage value is higher than the upper limit voltage value, and whether the driving current value of the blowing fan 700 is higher than the upper limit current value (S401, S402).

When the output voltage value is higher than the upper limit voltage value, and the driving current value is higher than the upper limit current value (S401: Yes->S402: Yes), the control part 910 starts timing through a timer (S403). Then, when the timer times to a specified duration, such as 5 seconds (S404: Yes), i.e., when a state that the output voltage value is higher than the upper limit voltage value and the driving current value is higher than the upper limit current value at least lasts for the specified duration, the control part 910 sets the damage mark as an ON state (S405). Before the timer times to the specified duration, when the output voltage value is less than the upper limit voltage value or the driving current value is less than the upper limit current value, the control part 910 resets the timer (S406).

In this way, under a condition that a series of wind paths are damaged, the damage mark is set as an ON state through treatment of judging damage of the wind paths.

By returning to Fig. 13 again, when treatment of judging blocking of wind paths and treatment of judging damage of wind paths are performed, next the control part 910 judges whether the blocking mark or the damage mark is in the ON state (S110). Under a condition that the blocking mark and the damage mark are in an OFF state (S110: No), the control part 910 judges whether operation end time is reached (S111). When the operation end time is not reached, the control part 910 returns to step S105, and repeatedly performs a series of treatments from stable control of the rotating speed of the fan (S105) to the judgment of the state of the blocking mark or the damage mark (S110).

On the other hand, when the blocking mark or the damage mark is set as the ON state (S110: Yes), the control part 910 controls the generator driving part 950 to stop the ozone generator 600 (S112). Then, the control part 910 enables the operation of the blowing fan 700 to last for the operation end time in a state of keeping stop of the ozone generator 600. During this period, since the ozone generator 600 is stopped, treatment of judging blocking of the wind paths and treatment of judging damage of the wind paths are not performed.

When it is judged that the operation end time is reached in step S111 (S111: Yes), the control part 910 controls the fan driving part 960 to stop the blowing fan 700. Moreover, when the ozone generator 600 is under operation, the generator driving part 950 is controlled to stop the ozone generator 600 (S113).

It should be noted that under a condition that the control part 910 already stops the ozone generator 600 and the blowing fan 700, the ozone generator 600 is initially stopped, and then after a specified waiting time elapses, for example, after 30 seconds elapse, the blowing fan 700 is stopped. In this control mode, since air without ozone is introduced into the bag body 10 and air with ozone is exhausted from the bag body 10 through the air, the air with ozone can be inhibited from remaining in the bag body 10 at the end of deodorization operation.

Next, the control part 910 judges whether the blocking mark is in the ON state (S114). When the blocking mark is not in the ON state (S114: No), the control part 910 judges whether the damage mark is in the ON state (S115). When the blocking mark is in the ON state (S114: Yes), the control part 910 controls the informing driving part 940 to light up the third informing part 456 (S116). When the damage mark is in the ON state (S115: Yes), the control part 910 controls the informing driving part 940 to light up the fourth informing part 457 (S117). In this way, control treatment of deodorization operation is ended.

It should be noted that under a condition that the deodorization button 452 is pressed in a state that the induction pipe 30 is not installed on the ozone supply device 20, the control part 910 judges that the induction pipe 30 is not installed on the ozone supply device 20 in step S101 (S101: No) and enables the first informing part 454 to light up (S118) and does not start deodorization operation. In addition, under a condition that the deodorization button 452 is pressed in a state that the throwing inlet 11 of the bag body 10 is not locked, the control part 910 judges that the throwing inlet 11 is not locked in step S102 (S102: No), and enables the second informing part 455 to light up (S119) and does not start deodorization operation.

### <Effects of Present Embodiment>

The following effect is performed above through the present embodiment.

(1) Since blocking of a series of wind paths of the clothes deodorizing device 1 can be detected, a measure corresponding to the blocking of the wind paths can be taken. Moreover, since the blocking of the wind paths can be detected through the blowing fan 700 included in the clothes deodorizing device 1 by monitoring a driving state of the blowing fan 700, i.e., an output voltage value and a driving current value, a special-purpose detecting unit for detecting the blocking of the wind paths is not required to be arranged, thereby inhibiting increase of a number of components and increase of cost.

(2) Since the wind paths are regarded as being blocked under a condition that both of the output voltage value and the driving current value of the blowing fan 700 are less than respective lower limit values, it is difficult to wrongly judge blocking of the wind paths due to influences of noise and the like. Further, since the wind paths are not immediately regarded as being blocked even if both of the output voltage value and the driving current value of the blowing fan 700 are less than respective lower limit values, it is more difficult to wrongly judge blocking of the wind paths due to influences of noise and the like.

(3) Since the ozone generator 600 is stopped under a condition that the wind paths are blocked, ozone can be prevented from being continuously generated fruitlessly when it is difficult to perform right clothes deodorization.

(4) Since the third informing part 456 lights up under a condition that the wind paths are blocked, the blocking of the wind paths can be notified to the user.

(5) Since damage of a series of wind paths of the clothes deodorizing device 1 can be detected, a measure corresponding to the damage of the wind paths can be taken. Moreover, since the damage of the wind paths can be detected through the blowing fan 700 included in the clothes deodorizing device 1 by monitoring a driving state of the blowing fan 700, i.e., an output voltage value and a driving current value, a special-purpose detecting unit for detecting the damage of the wind paths is not required to be arranged, thereby inhibiting increase of a number of components and increase of cost.

(6) Since the wind paths are regarded as being damaged under a condition that both of the output voltage value and the driving current value of the blowing fan 700 are less than respective lower limit values, it is difficult to wrongly judge blocking of the wind paths due to influences of noise and the like. Further, since the wind paths are not immediately regarded as being damaged even if both of the output voltage value and the driving current value of the blowing fan 700 are greater than respective upper limit values, it is more difficult to wrongly judge damage of the wind paths due to influences of noise and the like.

(7) Since the ozone generator 600 is stopped under a condition that the wind paths are damaged, ozone can be prevented from being continuously generated fruitlessly when it is difficult to perform right clothes deodorization.

(8) Since the fourth informing part 457 lights up under a condition that the wind paths are damaged, the blocking of the wind paths can be notified to the user.

(9) Since deodorization operation is not started when locking of the throwing inlet 11 of the bag body 10 is not detected through the lock detection part 470, opening of the throwing inlet 11 can be misjudged as damage of the wind paths.

### <Change Embodiment>

Embodiments of the present invention are described above, but the present invention is not limited to the above-mentioned embodiments. In addition, various changes except for the above can also be made to embodiments of the present invention.

For example, in the above embodiment, under a condition that the control part 910 obtains both of the output voltage value and the driving current value, the output voltage value is lower than the lower limit voltage value, and the driving current value is lower than the lower limit current value, the blocking mark is set as an ON state. However, under a condition that the control part 910 only obtains the output voltage value and the output voltage value is lower than the lower limit voltage value, the blocking mark is set as an ON state. In addition, under a condition that the control part 910 only obtains the driving current value and the driving current value is lower than the lower limit current value, the blocking mark is set as an ON state.

In addition, in the above embodiment, under a condition that the blocking mark is in an ON state or the damage mark is in an ON state, in step S112, the control part 910 stops the ozone generator 600. However, the control part 910 can also reduce the output of the ozone generator 600 in step S112.

Further, in the above embodiment, even if the ozone generator 600 is stopped when the blocking mark or the damage mark is in the ON state until the operation end time elapses, the blowing fan 700 can also continue to operate and continue to perform deodorization operation. However, the ozone generator 600 and the blowing fan 700 can also be stopped when the blocking mark or the damage mark is in the ON state, to end deodorization operation. In this case, ideally, the blowing fan 700 is stopped after a specified duration from the beginning of stop of the ozone generator 600.

Further, in the above embodiment, under a condition that the wind paths are blocked and damaged, the blowing fan 700 is stopped, and after deodorization operation is ended, the third informing part 456 and the fourth informing part 457 light up. However, the third informing part 456 and the fourth informing part 457 can light up immediately in the process of deodorization operation after the ozone generator 600 is stopped when the blocking mark or the damage mark is in the ON state.

Further, in the above embodiment, the clothes are accommodated in the bag body 10. However, the accommodating part for accommodating the clothes is not limited to the bag body 10, and can also be a box-shaped accommodating warehouse formed by resin and metal.

In addition, various changes can be properly made to embodiments of the present invention within a scope of technical concepts shown in claims.

### List of reference numerals

10: Bag body (accommodating part); 11: Throwing inlet; 20: Ozone supply device (ozone supply part); 456: Third informing part (informing part); 457: Fourth informing part (informing part); 470: Lock detection part (detection part); 600: Ozone generator; 700: Blowing fan; 910: Control part.

## Claims

1. A clothes treating device, comprising:
an accommodating part for accommodating clothes;
an ozone supply part comprising an ozone generator and a blowing fan, wherein ozone generated by the ozone generator is delivered into the accommodating part through flow of air by means of operation of the blowing fan; and
a control part for controlling operation of the ozone generator and the blowing fan,
wherein the control part
is capable of changing an output voltage value output to the blowing fan in a manner of enabling the blowing fan to keep specified rotating speed, and
obtains at least one value of the output voltage value when controlling the blowing fan to keep the specified rotating speed and a value of driving current flowing through the blowing fan, and executes treatment corresponding to a judgment result under a condition of judging that the obtained value is less than a specified threshold corresponding to the value.

2. The clothes treating device according to claim 1, wherein
the control part obtains both of the output voltage value and the driving current value when controlling the blowing fan to keep the specified rotating speed, and executes treatment corresponding to a judgment result under a condition of judging that the obtained output voltage value is less than a first threshold and the obtained driving current value is less than a second threshold.

3. A clothes treating device, comprising:
an accommodating part for accommodating clothes;
an ozone supply part comprising an ozone generator and a blowing fan, wherein ozone generated by the ozone generator is delivered into the accommodating part through flow of air by means of operation of the blowing fan; and
a control part for controlling operation of the ozone generator and the blowing fan,
wherein the control part
is capable of changing an output voltage value output to the blowing fan in a manner of enabling the blowing fan to keep specified rotating speed, and
obtains at least one value of the output voltage value when controlling the blowing fan to keep the specified rotating speed and a value of driving current flowing to the blowing fan, and executes treatment corresponding to a judgment result under a condition of judging that the obtained value is greater than a specified threshold corresponding to the value.

4. The clothes treating device according to claim 3, wherein
the control part obtains both of the output voltage value and the driving current value when controlling the blowing fan to keep the specified rotating speed, and executes treatment corresponding to a judgment result under a condition of judging that the obtained output voltage value is greater than a first threshold and the obtained driving current value is greater than a second threshold.

5. The clothes treating device according to claim 3 or 4, further comprising a detection part for detecting whether a throwing inlet for clothes arranged in the accommodating part is locked; wherein
the control part enables the ozone generator and the blowing fan not to operate under a condition of not detecting that the throwing inlet is locked through the detection part.

6. The clothes treating device according to any one of claims 1-5, wherein
as a treatment corresponding to the judging result, the control part stops the ozone generator or reduces an output of the ozone generator.

7. The clothes treating device according to any one of claims 1-6, further comprising an informing part for informing to outside; wherein
as a treatment corresponding to the judging result, the control part enables the informing part to operate.
